(19) Europäisches Patentamt
European Patent Office
Office européen des brevets

(11) Publication number: **0 497 314 A1**

(12) **EUROPEAN PATENT APPLICATION**

(21) Application number: **92101469.2**

(51) Int. Cl.5: **A61K 31/505**

(22) Date of filing: **29.01.92**

(30) Priority: **31.01.91 US 648252**

(43) Date of publication of application:
**05.08.92 Bulletin 92/32**

(84) Designated Contracting States:
**AT BE CH DE DK ES FR GB GR IT LI LU MC NL PT SE**

(71) Applicant: **Bristol-Myers Squibb Company**
**345 Park Avenue**
**New York, N.Y. 10154(US)**

(72) Inventor: **Riblet, Leslie A.**
**145 Chestnut Hill Road**
**Killingworth, Connecticut 06419(US)**
Inventor: **McKinney, Gordon R.**
**4036 Fall Creek Drive**
**Evansville, Indiana 47711(US)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

(54) **Use of azapirones in attention deficit hyperactivity disorder.**

(57) Buspirone and other azapirones and their pharmaceutically acceptable salts are useful in attention deficit hyperactivity disorder.

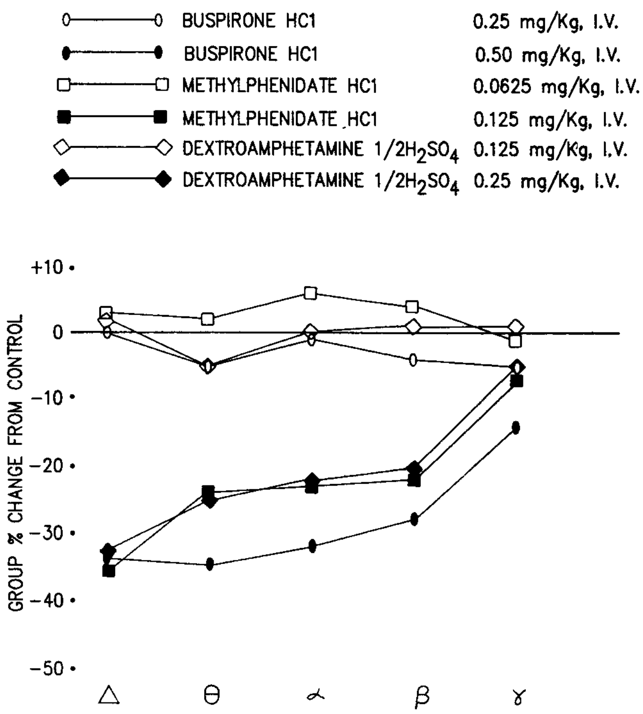


FIG.1

EP 0 497 314 A1

The present invention relates to buspirone and other azapirones and their pharmaceutically acceptable salts, for use in the treatment of attention deficit hyperactivity disorder.

Attention deficit hyperactivity disorder (ADHD) is a disorder of childhood that sometimes continues into adulthood. ADHD can be characterized by symptoms such as inattention, impulsivity and hyperactivity. There are indications that about one third of children with ADHD continue to show some signs of ADHD in adulthood. Richard Balon, J. Clin Psychopharmacol., Vol. 10, No. 1, p. 77 (Feb. 1990).

This invention is directed to a method of treatment of attention deficit hyperactivity disorder by the administration of the compound of the formula

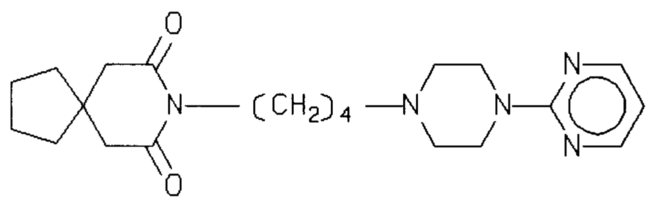

which is known as buspirone. The hydrochloride salt has been referred to in the prior art as MJ 9022-1 and buspirone hydrochloride. Other acid addition salts thereof are named by combining "buspirone" with the appropriate word to define the acid from which it is prepared as the "buspirone hydrochloride". The latter is the United States Adopted Name (USAN); refer to J. American Med. Assoc. 225, 520 (1973).

The synthesis of the compound and the disclosure of its psychotropic properties are described in the following patents and publications:

1. Y. H. Wu, et al, J. Med. Chem., 15,:477 (1972).
2. Y. H. Wu, et al., U. S. Pat. No. 3,717,634 which issued February 20, 1973.
3. L. E. Allen et al., Arzneium. Forsch. 24, No. 6, 917-922 (1974).
4. G. L. Sathananthan, et al., Current Therapeutic Research, 18/5, 701-705 (1975).
5. Y. H. Wu et al., U. S. Pat. No. 3,976,776, issued August 25, 1976.

The following patent references disclose and claim additional uses that relate to buspirone's pharmacological effects on the central nervous system.

1. The use of buspirone hydrochloride as a novel antianxiety agent for the treatment of neurotic patients is described in G. P. Casten, et al, U. S. Pat. No. 4,182,763, issued January 9, 1980.
2. Allen, et al, disclose the use of buspirone in treating extrapyramidal motor disorders in U.S. 4,438,119, issued March 20, 1984.
3. Buspirone's use in sexual dysfunction was described by Othmer, et al., in U.S. 4,640,921, issued February 3, 1987.
4. Kurtz, et al., in U. S. 4,634,703, issued January 6, 1987 disclose buspirone's use in treating panic disorders.
5. Alderdice discloses the use of buspirone in the improvement of short term memory in U.S. 4,687,772, issued August 18, 1987.
6. U.S. 4,777,173 of Shrotriya and Casten, issued October 11, 1988 discloses and claims the use of buspirone in treating alcohol abuse.

None of the above-referenced uses teach or suggest that buspirone and its pharmaceutically acceptable salts would be useful in the treatment of attention deficit hyperactivity disorder.

It is known that stimulants such as methylphenidate and dextroamphetamine and to a lesser extent the antipsychotics such as thioridazine and chlorpromazine are used clinically in the treatment of the childhood hyperkinetic syndrome often associated with minimal brain dysfunction. However, these are associated with various side effects such as delay in onset of sleep, decreased appetite, inhibition of growth, and mood changes.

Animal behavior studies indicate that buspirone possesses potential antipsychotic activity comparable to that of most phenothiazines but with minimal sedative effects Allen, et al, Pharmacologic Effects of MJ 9022-1, a Potential Tranquilizing Agent, Arzneim.-Forsch. (Drug Res.) 24: 917-922, 1974. In another study in which the effect of buspirone on evoked electrographic activity was determined in a limited number of cats, it was observed that buspirone possessed properties similar to both chlorpromazine and methamphetamine. John, et al, Factor Analysis of Evoked Potentials, Electroenceph. Clin. Neurophysiol. 34: 33-34, 1973.

A comparison of the effects of buspirone, methylphenidate, dextroamphetamine, thioridazine and chlorpromazine on the cortical EEG of mammals indicated that buspirone possesses stimulant properties

similar to that of methylphenidate and dextroamphetamine.

Because of this apparently unique combination of activities, a study was undertaken to compare the effects of these drugs on the cortical EEG of mammals with those produced by buspirone.

It was found that the ability of buspirone to generate an EEG profile characteristic of the stimulant drugs, in the absence of undesirable motor stimulation makes buspirone useful in the treatment of attention deficit hyperactivity disorder.

The method of the present invention is intended for the treatment of attention deficit hyperactivity disorder. The method essentially involves administration of buspirone, or a pharmacologically acceptable acid addition salt thereof, to a patient in need of such treatment.

Figure 1 shows the quantitative EEG profiles from the rabbit cortex.

Figure 2 shows the quantitative EEG profiles from the rabbit cortex.

Figure 3 shows the quantitative EEG profiles of buspirone HCl from the cat cortex.

Figure 4 shows the quantitative EEG profiles of methylphenidate and dextroamphetamine from the cat cortex.

Figure 5 shows the quantitative EEG profiles of thioridazine and chlorpromazine from the cat cortex.

The invention results from the discovery that administration of buspirone and its pharmaceutically acceptable salts is an effective treatment in preventing or reducing the incidence of attention deficit hyperactivity disorder.

Pharmaceutically acceptable acid addition salts of buspirone and methods of pharmaceutical formulation are described in the patents of Wu, et al., U.S. 3,717,634 and U.S. 3,976,776, which are incorporated in their entirety herein by reference.

Administration of buspirone and other azapirones according to the present invention may be made by the parenteral, oral, or rectal routes. Parenteral administration comprises injection, e.g, intravenous or intramuscular injection, as well as any other parenteral route of administration. In the use for children, the compound of the present invention can be mixed with a liquid base comprised of liquid lactose and artificial coloring to produce a syrup-like compound which can be administered to children in a liquid form. This liquid base can be flavored with various fruit flavors, such as cherry, to make the administration of the drug easier. Further, the compound of the present invention can be blended with powdered lactose and artificial coloring and formed into chewable tablets which can be taken orally by children in an form easier than in the normal unflavored tablet form. The clinical dosage range for treatment of ADHD will be from 1 to 60 mg per day depending on age of recipient, body weight, general physical condition and severity of disease. Dosage will be appropriately reduced for infants and young children, according to the clinical judgement of the attending physician.

## Methods

Quantitative EEG techniques were utilized to demonstrate that buspirone elicits an EEG profile similar to that produced by stimulant drugs, for example, methylphenidate and dextroamphetamine and dissimilar to that produced by thioridazine and chlorpromazine when administered intravenously to rabbits. However, when administered subcutaneously to cats high doses of buspirone resulted in an EEG profile which possesses attributes of both the antipsychotic and stimulant drugs.

(a) Adult Dutch-belted female rabbits, weighing 2.0-2.4 kg, were implanted with stainless steel recording electrodes over the central cortex. At least 2 weeks were allowed for recovery before beginning experimentation.

Groups of four animals each were individually restrained and connected to an EEG amplifier and frequency analyzer. The EEG of each animal was recorded on a separate channel. The instrumentation filtered each channel of EEG analog data into five distinct frequency bands: $\Delta = 0.5\text{-}3.5$ Hz, $\theta = 3.5\text{-}7.0$ Hz, $\alpha = 7\text{-}13$ Hz, $\beta = 13\text{-}25$ Hz and $\gamma = 25\text{-}40$ Hz. The EEG analyzer then integrated the filtered waveforms and sent pulses to an associated computer at a rate proportional to the electrical energy of each band. Thus, one computer print-out provided a cortical EEG profile for the entire group of four rabbits.

Data from a one-hour recording period were collected in four 15-minute periods, which provided additional information concerning both onset and time of peak effect of drug activity. However, in this report only the mean activity of the four data collection periods was considered since onset of drug activity was immediate and duration of action was at least one hour for all drugs studied. Drug-induced EEG changes were determined by comparing the post-treatment print-out to the pretreatment control in the same animal and statistical analysis was performed on change from control, in counts, using multiple Student-t paired-comparison tests with $p \leqq 0.05$ (Table 1). However, for clarity data are also presented as percent change from control (Figures 1 and 2).

All drugs were administered intravenously in saline solution as their respective salts and all dosage calculations were based on the free base. Recording was started immediately after injection and animals were not used more often than once per week. Initial doses were reduced geometrically until a no-effect dose was determined for each drug. However, only the "no-effect" data and the data generated from the next higher dose are presented in this report since the shape of the EEG profile is not dose-related, as opposed to the magnitude of the response which is dose-related.

Table 1 represents quantitative EEG data from the rabbit cortex. These data are drug induced changes resulting from the minimum dose of each compound required to elicit the EEG profile characteristic of that particular drug.

Figure 1 shows that in all cases, the stimulant profile is distinctly different from those produced by other classes of drugs. Therefore, based on these data one must assume that buspirone shares a component of stimulation similar to that of methylphenidate and dextroamphetamine.

Figure 2 represents the EEG profile of thioridazine and chlorpromazine which is completely different from that produced by stimulant drugs and is shown only for comparative purposes. It has, however, been reported that thioridazine and dextroamphetamine produce similar EEG profiles when studied in hyperactive children (Itil and Simeon, Computerized EEG in the Prediction of Outcome of Drug Treatment in Hyperactive Childhood Behavior Disorders, Psychopharmacology Bulletin 10(4): 36, 1974), which may explain the clinical utility of thioridazine in hyperkinesis.

By comparing minimal doses required to elicit EEG profiles characteristic of these drugs, these data indicate that methylphenidate is approximately four times more potent than buspirone. Dextroamphetamine, thioridazine and chlorpromazine are approximately twice as potent as buspirone. These data also indicate that buspirone is an exceptional compound in that it has an animal behavior profile similar to that of the phenothiazines on the one hand and an EEG profile characteristic of stimulant drugs on the other. This unique combination of activity may render buspirone useful in the treatment of childhood hyperkinesis which has been reported to respond to both selected phenothiazine and stimulant drugs.

(b) Adult mongrel female cats weighing 2.5-4.0 kg were implanted with EEG recording electrodes. Recording sites were as follows: fronto-occipital cortex, basal amygdala and ventral hippocampus.

Animals were individually held in canvas feline restraining bags for EEG recording. The EEG of each brain area was recorded on a separate channel. The instrumentation filtered each channel of EEG and analog data into five distinct frequency bands: $\Delta = 0.5\text{-}3.5$ Hz, $\theta = 3.5\text{-}7.0$ Hz, $\alpha = 7\text{-}13$ Hz, $\beta = 13\text{-}25$ Hz and $\gamma = 25\text{-}40$ Hz. The EEG analyzer then integrated the filtered waveforms and sent pulses to an associated computer at a rate proportional to the electrical energy of each band. Data collecting procedures were essentially the same as reported in Example 1. However, groups of six animals each were used and all drugs were administered subcutaneously in saline solution as their respective salts, but all dosages were calculated as the free base. Recording was started 30 minutes after injection to insure adequate drug absorption for peak activity.

Drug-induced EEG changes were determined by comparing the post-treatment data to the pre-treatment control in the same animal. Statistical analysis was performed on change from control, in instrument counts, using multiple Student-t-paired-comparison test with $P \leqq 0.05$ (Table 2). However, for clarity data are also presented as percent change from control (Figures 3, 4 and 5).

Table 2 represents quantitative EEG data from the fronto-occipital cortex of the cat. The drugs studied were without significant effect on electrical activity recorded from the amygdala and hippocampus and therefore these data are not included in this report. The data in Table 2 are drug-induced changes resulting from the minimum and higher doses of each compound required to elicit the EEG profile characteristic of that particular drug.

Figure 3 shows that the stimulant profile resulting from low doses (0.25 and 0.5 mg/kg, s.c.) of buspirone is similar to that produced by methylphenidate and dextroamphetamine administered over a wide dosage range (Figure 4). However, higher dose (2.0 and 8.0 mg/kg, s.c.) of buspirone (Figure 3) elicit a profile which deviates from that produced by the stimulants. Moreover, the 8.0 mg/kg dose of buspirone causes an increase in electrical "power" in the lowest frequency bands which is characteristic of antipsychotic compounds such as thioridazine and chlorpromazine (Figure 5).

This combination of activity, low-dose stimulant profile which changes at higher doses to include activity associated with both antipsychotic and stimulant drugs, makes buspirone a unique drug. This dual activity may render buspirone useful in the treatment of childhood hyperkinesis which has been reported to respond to both selected phenothiazines and stimulants. Itil and Simeon, Computerized EEG in the Prediction of Outcome of Drug Treatment in Hyperactive Childhood Behavior Disorders, Psychopharmacology Bulletin 10 (4): 36, 1974).

By comparing minimal doses required to elicit EEG profiles characteristic of these drugs, these data

indicate that dextroamphetamine is approximately twice as potent as buspirone and methylphenidate, which are of equal potency. In addition, buspirone is approximately four times more potent than thioridazine and chlorpromazine based on the minimal effective dose required to alter the EEG. However doses of buspirone at least twice that of thioridazine and chlorpromazine are required to produce the combined antipsychotic-stimulant EEG profile.

Table 1. Quantitative EEG Data from the Rabbit Cortex

| Treatment | Dose of free base mg/kg, i.v. | Δ | θ | α | β | γ |
|---|---|---|---|---|---|---|
| Buspirone HCl<br>" | 0.25†<br>0.50‡ | -1 ± 18 (0)<br>-114 ± 25* (-34) | -10 ± 14 (-5)<br>-86 ± 11* (-35) | -2 ± 12 (-1)<br>-70 ± 13* (-32) | -5 ± 7 (-4)<br>-47 ± 10* (-28) | -5 ± 2 (-5)<br>-17 ± 3* (-14) |
| Methylphenidate HCl<br>" | 0.0625†<br>0.125± | +7 ± 19 (+3)<br>-120 ± 36* (-36) | +3 ± 9 (+6)<br>-60 ± 16* (-24) | +8 ± 10 (+6)<br>-47 ± 16 (-23) | +4 ± 6 (+4)<br>-35 ± 6* (-22) | -1 ± 2 (-1)<br>-9 ± 2* (-7) |
| Dextroamphetamine 1/2$H_2SO_4$<br>" | 0.125†<br>0.25‡ | +4 ± 17 (+2)<br>-64 ± 19* (-33) | -9 ± 6 (-5)<br>-35 ± 9* (-25) | 0 ± 9 (0)<br>+27 ± 9* (-22) | +2 ± 6 (+1)<br>-21 ± 6* (-20) | +1 ± 1 (+1)<br>-4 ± 1* (-5) |
| Thioridazine HCl<br>" | 0.125†<br>0.25‡ | +11 ± 23 (+3)<br>+143 ± 11* (+69) | -13 ± 8 (-5)<br>+43 ± 4* (+21) | 0 ± 6 (0)<br>+54 ± 2* (+34) | +5 ± 4 (+3)<br>+39 ± 4* (+35) | +1 ± 1 (+1)<br>+5 ± 1* (+5) |
| Chlorpromazine HCl<br>" | 0.125†<br>0.25‡ | +30 ± 17 (+15)<br>+198 ± 51* (+75) | +20 ± 17 (+13)<br>+65 ± 22 (+33) | +21 ± 19 (+14)<br>+65 ± 18* (+42) | +12 ± 11 (+10)<br>+42 ± 13* (+30) | -3 ± 2 (-3)<br>+4 ± 1* (+5) |

Data are mean changes ± S.E. in electrical energy from pre-drug controls as actual instrument counts using 4 animals per treatment except **where n=6.

Group percent changes are shown in ( ) and are also represented graphically in Figures 1 and 2.

* = significant ($p \leq 0.05$) changes from control,

† = the no-effect dose

‡ = the minimum dose required to produce the EEG profile characteristic of that particular drug. Frequency bands are designated as

Δ = 0.5-3.5 Hz, θ= 3.5-7.0 Hz, α= 7-13 Hz, β= 13-25 Hz and γ= 25-40 Hz.

Table 2. Quantitative EEG Data from the Fronto-Occipital Cat Cortex

| Treatment | Dose of free base mg/kg, s.c. | Δ | θ | α | β | γ |
|---|---|---|---|---|---|---|
| Buspirone HCl | 0.125† | -46 ± 27 (-21) | -32 ± 16 (-22) | -23 ± 12 (-18) | -6 ± 4 (-6) | +4 ± 3 (+5) |
| | 0.25‡ | +10 ± 8 (+5) | -21 ± 6* (-16) | -35 ± 6* (-26) | -24 ± 5* (-17) | -1 ± 3 (0) |
| | 0.5 | -31 ± 18 (-14) | -46 ± 14*(-32) | -47 ± 12*(-39) | -24 ± 3* (-23) | -4 ± 4 (-5) |
| | 2.0 | +30 ± 22 (+15) | +17 ± 15 (+13) | -20 ± 14 (-15) | -33 ± 8* (-24) | -10 ± 3* (-9) |
| | 8.0 | +89 ± 29* (+55) | +35 ± 15 (+34) | -5 ± 14 (-6) | -22 ± 7* (-21) | -2 ± 3 (-3) |
| Methylphenidate HCl | 0.125† | -44 ± 18 (-22) | -31 ± 11* (-20) | -24 ± 10 (-18) | -3 ± 5 (-2) | +6 ± 2 (+7) |
| | 0.25‡ | -31 ± 18 (-14) | -38 ± 16 (-27) | -39 ± 12* (-31) | -11 ± 4* (-9) | +7 ± 2* (+9) |
| | 1.0 | -4 ± 30 (-2) | -45 ± 17* (-34) | -52 ± 14* (-46) | -20 ± 5* (-19) | +10 ± 3* (+13) |
| | 4.0 | -16 ± 45 (-8) | -56 ± 14* (-41) | -63 ± 11* (-51) | -14 ± 4* (-12) | +25 ± 5* (+35) |
| Dextroamphetamine 1/2$H_2SO_4$ | 0.0625† | -18 ± 6* (-8) | -15 ± 7 (-10) | -12 ± 8 (-8) | +1 ± 5 (+1) | +2 ± 3 (+2) |
| | 0.125‡ | -85 ± 21* (-35) | -44 ± 8* (-33) | -29 ± 9* (-26) | -3 ± 4 (-3) | +8 ± 2* (+12) |
| | 0.5 | -78 ± 32 (-33) | -76 ± 13* (-51) | -71 ± 11* (-51) | -18 ± 9 (-15) | +18 ± 4* (+25) |
| | 4.0 | +64 ± 27 (+29) | -34 ± 13 (-24) | -44 ± 13* (-35) | -6 ± 9 (-5) | +50 ± 8* (+63) |
| Thioridazine HCl | 0.5† | +11 ± 21 (+5) | -2 ± 8 (-1) | 0 ± 7 (0) | -3 ± 3 (-3) | -3 ± 3 (-5) |
| | 1.0‡ | +38 ± 11* (+19) | +29 ± 5* (+24) | +20 ± 5* (+18) | +3 ± 4 (+3) | -9 ± 4 (-11) |
| | 8.0 | +54 ± 8* (+28) | +40 ± 6* (+30) | +25 ± 6* (+26) | -3 ± 4 (-2) | -12 ± 3* (-15) |
| Chlorpromazine HCl | 0.5† | +14 ± 23 (+6) | +22 ± 15 (+15) | +23 ± 14 (+19) | +6 ± 5 (+6) | -10 ± 4 (-13) |
| | 1.0‡ | +46 ± 16* (+25) | +28 ± 10*(+20) | +33 ± 8* (+25) | +13 ± 6 (+9) | -10 ± 5 (-10) |
| | 4.0 | +83 ± 13* (+56) | +49*± 7* (+53) | +33 ± 2* (+38) | -3 ± 5 (-3) | -11 ± 3* (-14) |

Data are mean changes ± S.E. in electrical energy from pre-drug controls as actual instrument counts using 6 animals per treatment. Group percent changes are shown in ( ) and are also represented graphically in Figures 3, 4, and 5.

* = significant ($p \leq 0.05$) changes from control,

† = the no-effect dose

‡ = the minimum dose required to produce the EEG profile characteristic of that particular drug. Frequency bands are designated as Δ = 0.05-35 Hz, θ= 3.5-7.0 Hz, α= 7-13 Hz, β= 13-25 Hz and γ= 25-40 Hz.

The present invention is illustrated by the following examples which are not intended to be construed as limiting the scope of the invention.

EXAMPLE 1

Buspirone is administered to an eight year old, who has been identified by a clinician as having the symptoms associated with attention deficit hyperactivity disorder. During treatment, the child received 5 mg/kg of buspirone twice a day.

During and after treatment, the child showed spectacular and very fast improvement. The child became more attentive, impulsiveness decrease, and had no sleep disturbance.

EXAMPLE 2

Buspirone is administered to an eleven year old, who has been identified by a clinician as having the symptoms associated with attention deficit hyperactivity disorder. During the treatment, the child received 5 mg/kg of buspirone three times a day.

During and after treatment, the child showed excellent response.

## Claims

**1.** The use of at least one azapirone or a pharmaceutically effective acid addition salt thereof for the manufacture of a medicament for the treatment of attention deficit disorder with hyperactivity.

**2.** The use as described in claim 1 in which the azapirone is buspirone.

**3.** The use as described in claim 1 or 2 in which the medicament is combined with a liquid lactose to produce a syrup-like compound for oral administration.

**4.** The use as described in claim 1 or 2 in which the medicament is blended with powdered lactose to produce a chewable tablet for oral administration.

**5.** The use as described in any one of claims 1 to 4 in which the dosage is limited to between about 0.025 mg per pound of weight up to a maximum of 60 mg per day.

**6.** A pharmaceutical formulation for use in the treatment of attention deficit disorder with hyperactivity which comprises at least one azapirone or a pharmaceutically effective acid addition salt thereof in combination with pharmaceutically acceptable carrier.

**7.** The formulation as claimed in claim 6 in which the azapirone is buspirone.

**8.** The formulation as claimed in claim 6 or 7 which additionally comprises a liquid lactose to produce a syrup-like composition for oral administration.

**9.** The formulation as claimed in claim 6 or 7 in which the ingredients are blended with powdered lactose to produce a chewable tablet for oral administration.

**10.** A process for producing the formulation of claims 6 to 9 which comprises incorporating at least one azapirone or a pharmaceutically effective acid addition salt thereof into a suitable pharmaceutical acceptable carrier.

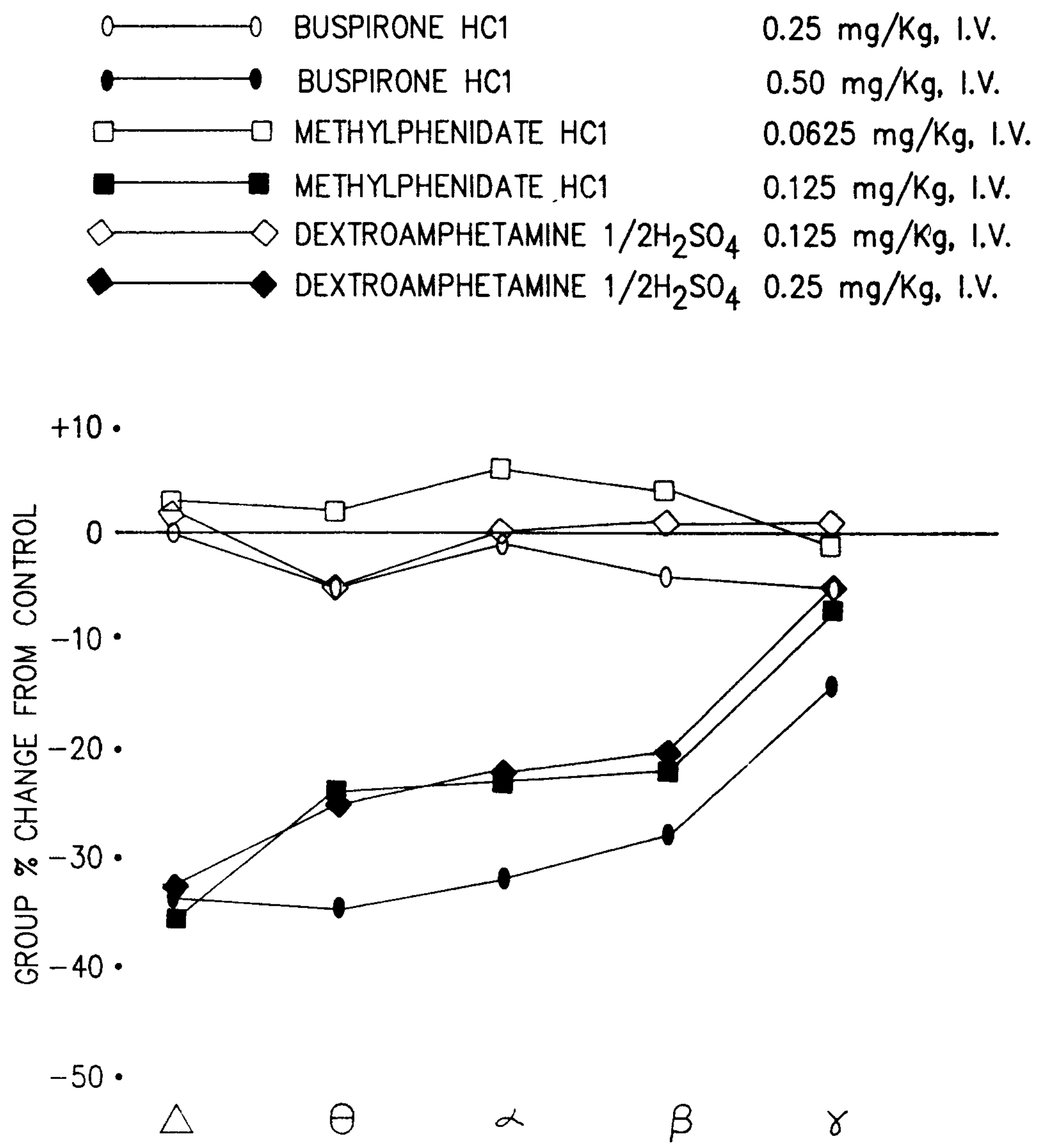
BUSPIRONE HC1 0.25 mg/Kg, I.V.
BUSPIRONE HC1 0.50 mg/Kg, I.V.
METHYLPHENIDATE HC1 0.0625 mg/Kg, I.V.
METHYLPHENIDATE HC1 0.125 mg/Kg, I.V.
DEXTROAMPHETAMINE 1/2H2SO4 0.125 mg/Kg, I.V.
DEXTROAMPHETAMINE 1/2H2SO4 0.25 mg/Kg, I.V.
GROUP % CHANGE FROM CONTROL
+10
0
-10
-20
-30
-40
-50
Δ
θ
α
β
γ

FIG.1

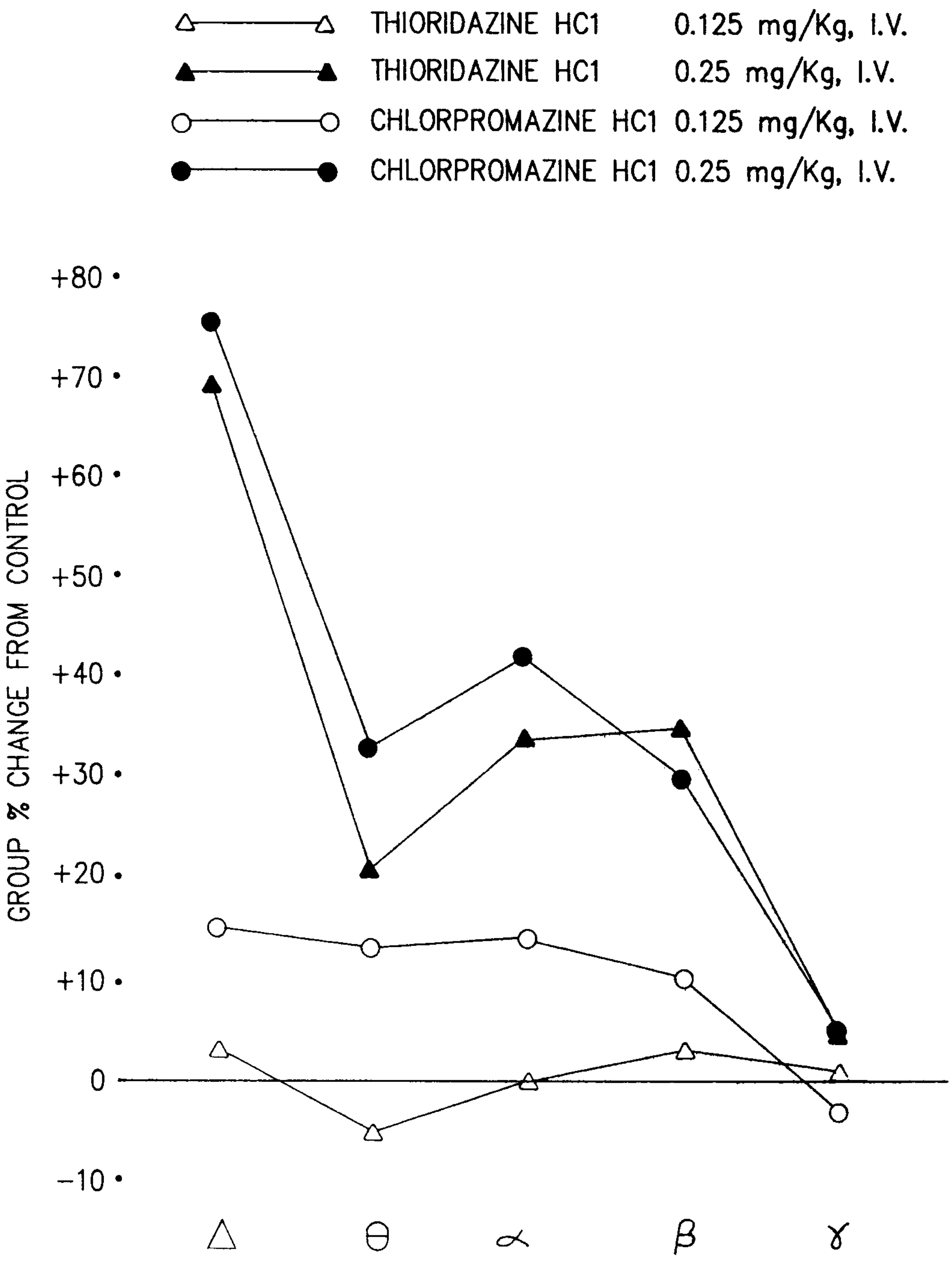
THIORIDAZINE HC1 0.125 mg/Kg, I.V.
THIORIDAZINE HC1 0.25 mg/Kg, I.V.
CHLORPROMAZINE HC1 0.125 mg/Kg, I.V.
CHLORPROMAZINE HC1 0.25 mg/Kg, I.V.
GROUP % CHANGE FROM CONTROL
+80
+70
+60
+50
+40
+30
+20
+10
0
−10
Δ
θ
α
β
γ

FIG.2

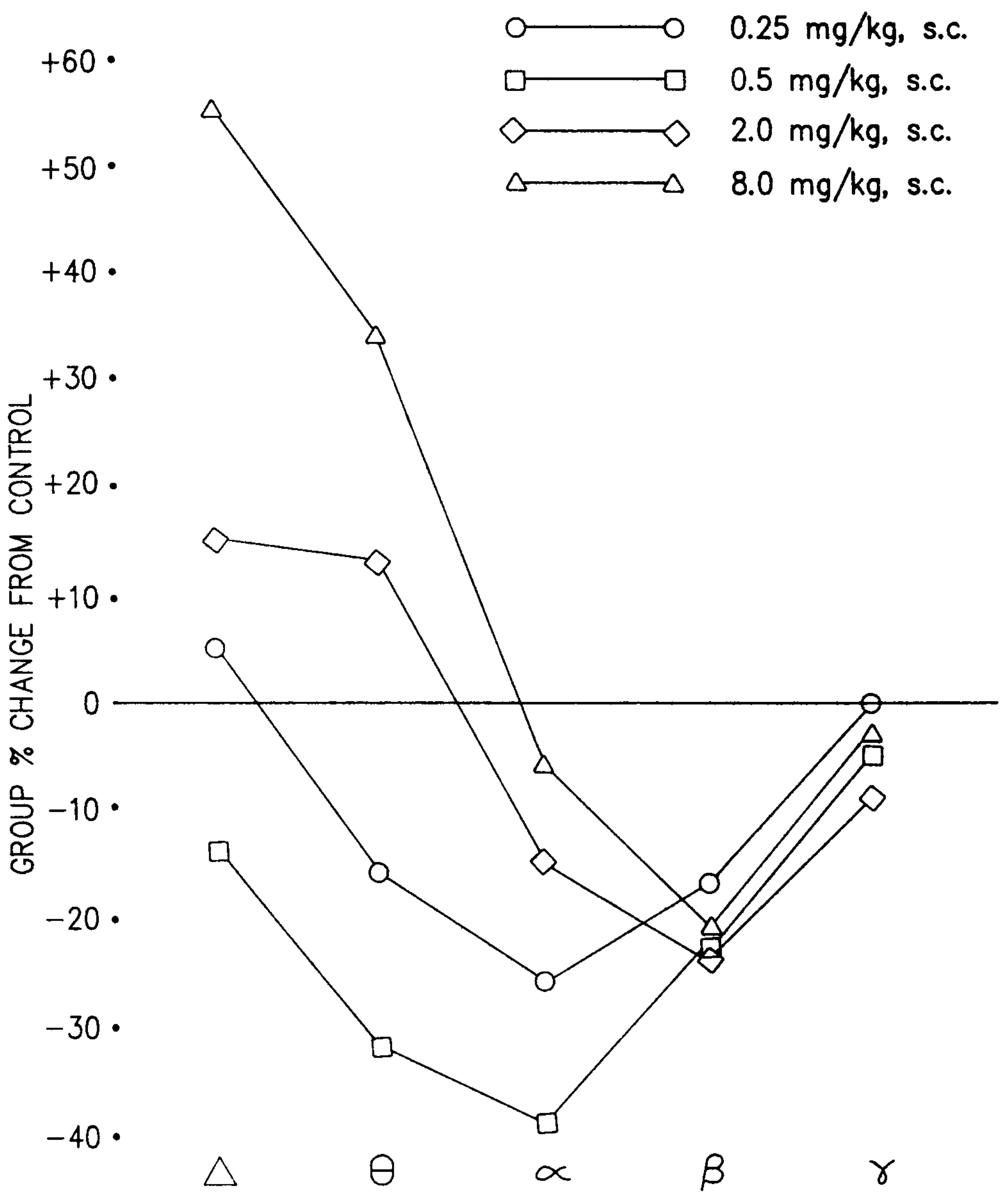
0.25 mg/kg, s.c.
0.5 mg/kg, s.c.
2.0 mg/kg, s.c.
8.0 mg/kg, s.c.
GROUP % CHANGE FROM CONTROL
+60
+50
+40
+30
+20
+10
0
−10
−20
−30
−40
Δ
θ
α
β
γ

FIG.3

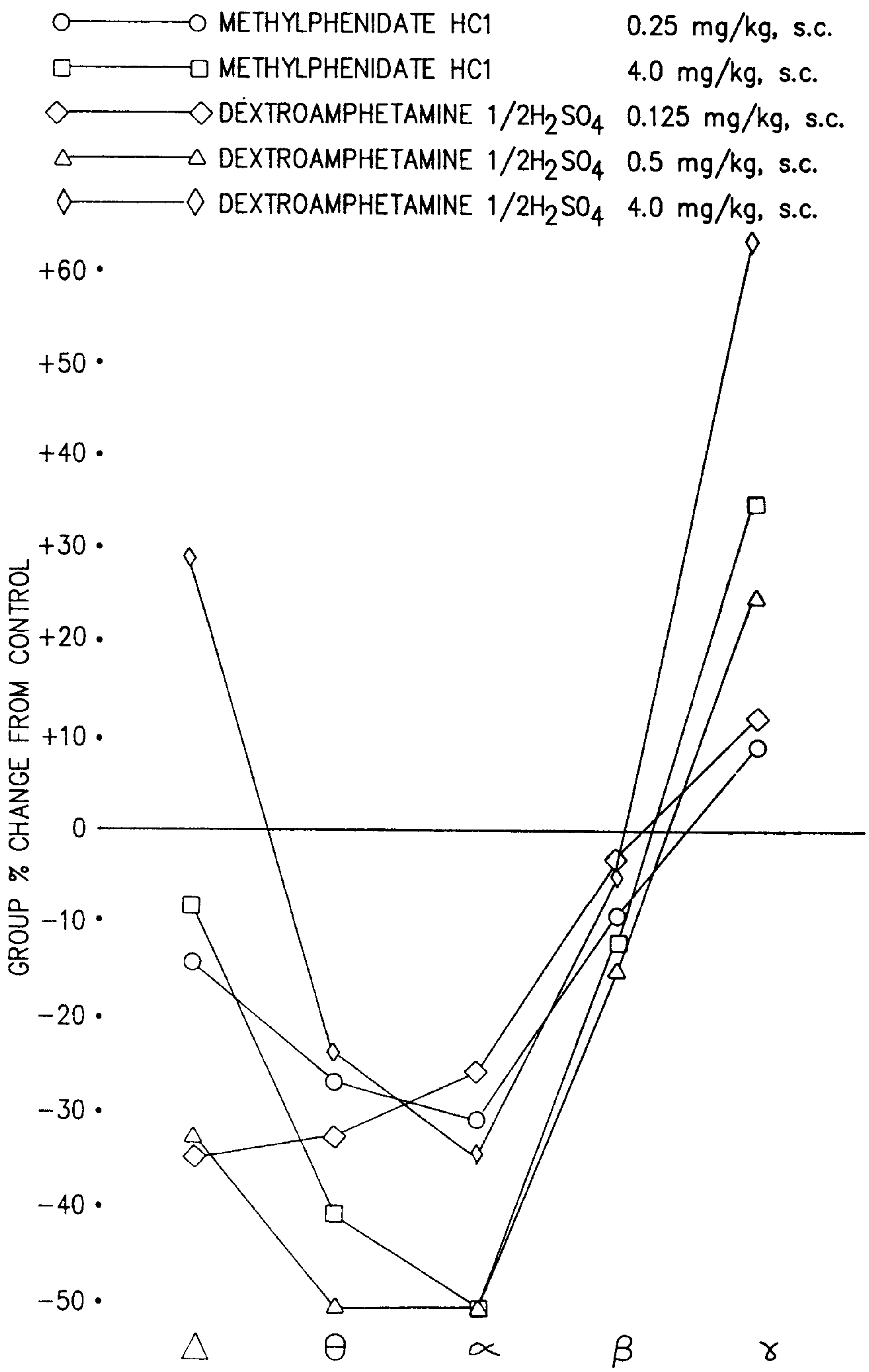
METHYLPHENIDATE HC1 0.25 mg/kg, s.c.
METHYLPHENIDATE HC1 4.0 mg/kg, s.c.
DEXTROAMPHETAMINE 1/2$H_2SO_4$ 0.125 mg/kg, s.c.
DEXTROAMPHETAMINE 1/2$H_2SO_4$ 0.5 mg/kg, s.c.
DEXTROAMPHETAMINE 1/2$H_2SO_4$ 4.0 mg/kg, s.c.
+60
+50
+40
+30
+20
+10
0
-10
-20
-30
-40
-50
GROUP % CHANGE FROM CONTROL
Δ
θ
α
β
γ

FIG.4

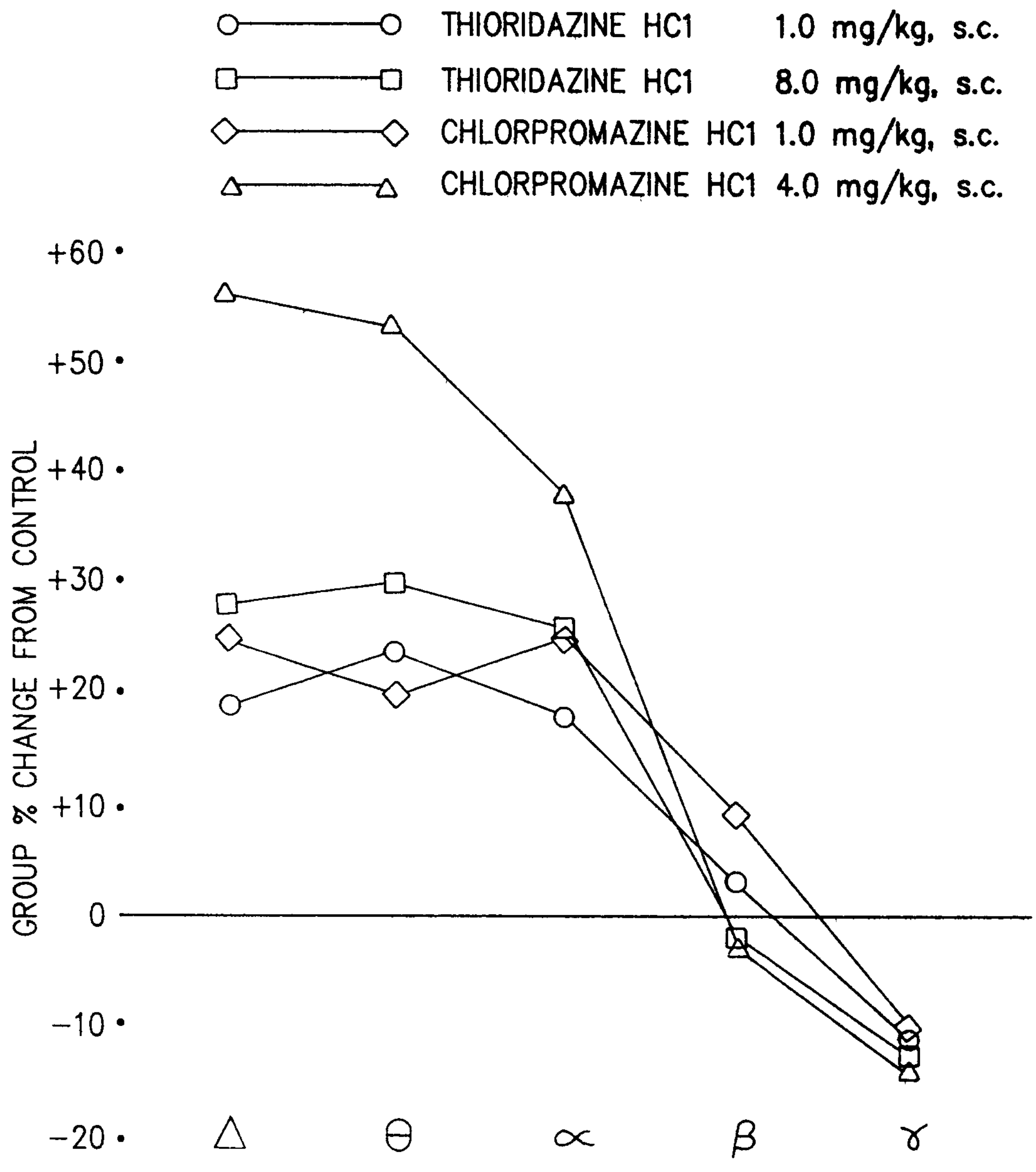
THIORIDAZINE HC1 1.0 mg/kg, s.c.
THIORIDAZINE HC1 8.0 mg/kg, s.c.
CHLORPROMAZINE HC1 1.0 mg/kg, s.c.
CHLORPROMAZINE HC1 4.0 mg/kg, s.c.
GROUP % CHANGE FROM CONTROL
+60
+50
+40
+30
+20
+10
0
-10
-20
Δ
θ
α
β
γ

FIG.5

European Patent Office

# EUROPEAN SEARCH REPORT

Application Number

EP 92 10 1469

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | J. CLIN. PSYCHOPHARMACOL. vol. 10, no. 1, February 1990, page 77; R. BALON: 'Buspirone for Attention Deficit Disorder' | 1,2,5 | A61K31/505 |
| Y | * the whole document * | 3,4 | |
| X | J. CLIN. PSYCHOPHARMACOL. vol. 9, no. 2, April 1989, pages 122 - 125; G. REALMUTO ET. AL.: 'Clinical Effect of Buspirone in Autistic Children' * abstract; page 123, i.e. case 1 and 2* | | |
| P,X | PEDIATR. CLIN. NORTH AM. vol. 38, no. 3, June 1991, pages 607 - 635; A.L. ROSTAIN: 'Attention deficit disorders in children and adolescents' * page 622, paragraph 2 * | 1-5 | |
| | | | TECHNICAL FIELDS SEARCHED (Int. Cl.5) |
| X | EP-A-0 220 696 (BRISTOL-MEYERS CO.) | 6,7,10 | |
| Y | * page 7, line 26 - page 8; claims 2,6 * | 8,9 | |
| D | & US-A-4 634 703 | | A61K |
| Y | '15th edition: Remington's Pharmaceutical Sciences' 1975 , MACK PUBLISHING CO. , EASTON, PENNSYLVANIA, USA *page 1263, Lactose USP* | 3,4,8,9 | |
| A | PSYCHOPATHOLOGY vol. 22, no. SUP1, 1989, pages 13 - 20; M.S. EISON: 'The new generation of seotonergic Anxiolytics: Possible clinical roles' * the whole document * | 1-10 | |

The present search report has been drawn up for all claims

| Place of search | Date of completion of the search | Examiner |
|---|---|---|
| MUNICH | 06 APRIL 1992 | FOERSTER W.K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document